# EUROPÄISCHE PATENTSCHRIFT

(11) **EP 2 918 220 B1**
(45) Veröffentlichungstag und Bekanntmachung des Hinweises auf die Patenterteilung: **30.08.2017**
(21) Anmeldenummer: 14158588.5
(22) Anmeldetag: 10.03.2014
(51) Int. Cl.: A61B 1/12, A61B 90/70

(54) **Bürste zur Innenreinigung eines flexiblen Katheters**
Brush for cleaning the interior of a flexible catheter
Brosse pour le nettoyage intérieur d'un cathéter flexible

(43) Veröffentlichungstag der Anmeldung: 16.09.2015
(73) Patentinhaber: MyBrush GmbH, 91572 Bechhofen (DE)
(72) Erfinder: Schellenberger, Gisela, 91572 Bechhofen a. d. Heide (DE)
(74) Vertreter: Dr. Gassner & Partner mbB

(56) Entgegenhaltungen:
- US-A- 5 168 593
- US-A- 5 297 310
- US-A- 5 615 439
- US-A1- 2001 016 962
- US-A1- 2004 187 892
- US-A1- 2011 028 787

## Beschreibung

Die Erfindung betrifft eine Bürste zur Innenreinigung eines flexiblen Katheters, wobei die Bürste einen Bürstenkopf und einen länglichen flexiblen Bürstenkopfhalter zum Vorschieben und Zurückziehen des Bürstenkopfes in dem Katheter aufweist. Der Bürstenkopf umfasst dabei einen gedrehten Haltedraht oder eine Mehrzahl gedrehter Haltedrähte und Fasern, wobei die Fasern von dem Haltedraht oder den Haltedrähten gehalten werden und einen Bürstenbesatz bilden. Die Fasern können aus einem Kunststoff oder einem natürlich vorkommenden Material, wie z. B. Borsten, bestehen.

Bürsten mit von einem gedrehten Haltedraht/gedrehten Haltedrähten gehaltenen Fasern, die einen Bürstenbesatz bilden, und einem länglichen flexiblen Bürstenkopfhalter zum Vorschieben und Zurückziehen des Bürstenkopfes sind im Stand der Technik bekannt. Zur Herstellung des Bürstenbesatzes werden die Fasern entweder zwischen zwei Haltedrähten oder zwischen zwei Schenkeln eines gefalzten Haltedrahtes angeordnet. Dann wird der Haltedraht verdreht. Über den dadurch gebildeten Bürstenbesatz der Bürste hinausgehender Haltedraht bzw. hinausgehende Haltedrähte wird/werden ebenfalls verdreht und bildet/bilden dadurch einen länglichen flexiblen Bürstenkopfhalter zum Vorschieben und Zurückziehen des Bürstenbesatzes. Derartige Bürsten sind beispielsweise im Bereich der Kessel- oder Rauchabzugsreinigung bekannt.

Zum Reinigen von Biopsieentnahmekathetern in medizinischen Endoskopen werden von der Firma MICRO-TECH Europe GmbH, Düsseldorf, Deutschland, Reinigungsbürsten mit je einem Bürstenkopf angeboten. Um zu verhindern, dass ein Arbeitskanal des Endoskops während der Reinigung beschädigt wird, ist am Bürstenkopfende ein Kugelköpfchen vorgesehen. Der Bürstenkopf mit dem gedrehten Haltedraht ist dabei auf einen Kunststoffschlauch aufgesetzt und an diesem fixiert. Am Ende dieses Kunststoffschlauchs ist ein Haltegriff zur besseren Handhabung angeordnet. Derartige Bürsten sind zur Reinigung von Kathetern mit einem Durchmesser von 1,3 mm geeignet. Problematisch ist dabei, dass sich dieses System nicht an kleinere Katheterdurchmesser anpassen lässt, weil der Durchmesser des Bürstenbesatzes des Bürstenkopfes zur Erzielung einer Reinigungswirkung stets größer als der äußere Schlauchdurchmesser sein muss und der Schlauchdurchmesser nicht beliebig dünn gewählt werden kann. Durch einen dünneren Schlauch ist keine gute Kraftübertragung mehr möglich und darüber hinaus besteht die Gefahr des Reißens des Schlauchs beim Zurückziehen des Bürstenkopfs.

Gemäß der DE 699 37 496 T2 weist eine übliche Endoskopreinigungsbürste einen langen flexiblen Edelstahlkörper auf, der aus einem flexiblen Edelstahldrahtkern besteht, der mit eng gewickelten flexiblen Edelstahlwicklungen bedeckt ist. Der Durchmesser des Edelstahlkörpers beträgt ungefähr 1 mm. Am Ende des Körpers befindet sich ein Abschnitt aus flexiblen NylonFasern. Diese Fasern sind üblicherweise mit den Edelstahlwicklungen verflochten. Der Bürstenabschnitt weist einen Durchmesser von ungefähr 5 - 6 mm auf. Die Reinigungsbürste wird verwendet, um entlang der gesamten Länge des Lumens eines Endoskops schnell vor und zurück zu bürsten, um eine Höchstmenge an biologischer Materialverunreinigung zu entfernen.

Aufgabe der vorliegenden Erfindung ist es, eine Bürste anzugeben, die so ausgebildet werden kann, dass sie zur Reinigung eines Katheters mit einem Innendurchmesser von weniger als 1,2 mm geeignet ist.

Die Aufgabe wird durch die Merkmale des Patentanspruchs 1 gelöst. Zweckmäßige Ausgestaltungen ergeben sich aus den Merkmalen der Patentansprüche 2 bis 10.

Erfindungsgemäß ist eine Bürste zur Innenreinigung eines flexiblen Katheters vorgesehen, wobei die Bürste einen Bürstenkopf und einen länglichen flexiblen Bürstenkopfhalter zum Vorschieben und Zurückziehen des Bürstenkopfes in dem Katheter aufweist. Der Bürstenkopf umfasst dabei einen gedrehten Haltedraht oder eine Mehrzahl gedrehter Haltedrähte. "Mehrzahl" im Sinne der Erfindung bedeutet mindestens zwei. Weiterhin umfasst der Bürstenkopf Fasern, wobei die Fasern von dem Haltedraht oder den Haltedrähten gehalten werden und einen Bürstenbesatz bilden. Der Bürstenkopfhalter besteht aus nur einem flexiblen, ungedrehten Draht, an dessen einem Ende der Halterdraht oder die Haltedrähte stumpf angeschweißt ist/sind. Am anderen Ende des Bürstenkopfhalters kann ein Griff zur besseren Handhabung der Bürste angeordnet sein. Der Bürstenbesatz weist im Allgemeinen einen runden Querschnitt auf. Der Durchmesser dieses Querschnitts kann sich in Richtung der Stelle, an welcher der Haltedraht oder die Haltedrähte am Draht angeschweißt ist/sind, erhöhen, d. h. der Bürstenbesatz kann eine konische Form aufweisen.

Der Erfinder der vorliegenden Erfindung hat erkannt, dass sich die im Stand der Technik bekannten Bürsten zur Reinigung von Kathetern in Endoskopen nicht beliebig im Durchmesser verringern lassen, ohne dabei ihre Funktionsfähigkeit zu verlieren. Weiterhin hat er erkannt, dass beliebig verkleinerte bekannte Bürsten, bei denen sich der gedrehte Haltedraht jenseits der Fasern einfach fortsetzt, zur Reinigung von Kathetern in Endoskopen nicht geeignet sind, weil die Oberfläche des keinen Bürstenbesatz aufweisenden gedrehten Drahtes zu einer mechanischen Belastung der Innenwand des Katheters führt, so dass dabei die Gefahr von Beschädigungen besteht. Durch das Vorsehen nur eines flexiblen, ungedrehten und damit oberflächlich glattflächigen Drahtes als Bürstenkopfhalter besteht diese Gefahr nicht. Darüber hinaus kann durch den Draht auch bei sehr geringem Drahtdurchmesser noch genügend Kraft auf den Bürstenkopf übertragen werden, um diesen in dem Katheter vorschieben zu können und zurückziehen zu können, ohne dass die Gefahr des Abreißens des Bürstenkopfes besteht.

Das stumpfe Anschweißen des gedrehten Haltedrahtes oder der gedrehten Haltedrähte an dem flexiblen Draht kann mittels Laserstrahlung erfolgen. Das Zuführen von zusätzlichem Material, beispielsweise in Form eines Schweißdrahtes ist dabei nicht erforderlich. Das zum Schweißen erforderliche Material wird hier vorzugsweise vom Haltedraht bzw. den Haltedrähten und/oder vom flexiblen Draht selbst bereitgestellt. Durch den Laserstrahl wird lediglich ein Ende des flexiblen Drahtes mit einem Ende des Haltedrahtes bzw. mit der Mehrzahl der Enden der Haltedrähte verschmolzen. Mittels dieser Technik kann eine Zunahme des Durchmessers durch das Schweißen nahezu vollständig vermieden werden. Durch das stumpfe Verschweißen des Haltedrahtes/der Haltedrähte mit dem flexiblen Draht ist die Bürste sehr flexibel und kann dadurch auch durch stark gekrümmte Katheter geschoben werden. Eine durch das Verschweißen verhärtete Stelle ist dabei im Allgemeinen nur zwischen 0,5 und 1 mm lang. Bei einem alternativ denkbaren Verbinden des Drahtes mit dem Haltedraht/den Haltedrähten mittels einer den Draht und den Haltedraht/die Haltedrähte überdeckenden und mit dem Draht und dem Haltedraht/den Haltedrähten verpressten oder verschweißten Hülse wäre der unflexible Bereich etwa 5 mm lang, so dass mit einer solchen Bürste Katheter mit einem engen Krümmungsdurchmesser nicht gereinigt werden könnten. Darüber hinaus würde durch eine unflexible Hülse eine erhöhte Beschädigungsgefahr für das Lumen des Katheters bestehen.

Ein weiterer Vorteil im medizinischen Bereich besteht darin, dass der flexible Draht im Gegensatz zum im Stand der Technik üblichen Schlauch chemisch relativ beständig ist und keine Hohlräume aufweist. Er lässt sich dadurch mit üblichen, teilweise auch aggressiven, Desinfektionsmitteln und/oder hoher Temperatur einfach reinigen und desinfizieren. Im Stand der Technik bekannte Schläuche erfordern eine Desinfektion mit Gas oder Gammastrahlung. Grundsätzlich ist die erfindungsgemäße Bürste durch die problemlose Desinfektionsmöglichkeit ohne Weiteres auch mehrfach verwendbar. Eine Verbindung des Haltedrahts/der Haltedrähte mit dem Draht mittels der oben genannten Hülse wäre auch bei einer Wiederverwendung problematisch, da durch Kapillarkräfte in dem gedrehten Haltedraht/den gedrehten Haltedrähten organisches Material in die Hülse eindringen und sich dort halten kann. Eine Wiederverwendbarkeit wäre dadurch nahezu ausgeschlossen.

Bei einer Ausgestaltung der erfindungsgemäßen Bürste ist ein Übergang vom Haltedraht/von den Haltedrähten zum Draht gratfrei. Bei dieser oder einer anderen Ausgestaltung der erfindungsgemäßen Bürste weicht ein maximaler Gesamtau-βendurchmesser eines gedrehten Bereichs des gedrehten Haltedrahtes/der gedrehten Haltedrähte vom Außendurchmesser des Drahtes gar nicht oder um höchstens 0,1 mm, insbesondere höchstens 0,075 mm, insbesondere höchstens 0,05 mm, insbesondere höchstens 0,025 mm, insbesondere höchstens 0,01 mm, ab. Beim maximalen Gesamtaußendurchmesser des gedrehten Bereichs des gedrehten Haltedrahtes/der gedrehten Haltedrähte werden die Fasern des Bürstenbesatzes außer acht gelassen. Durch die genannte geringe oder nicht vorhandene Abweichung und/oder die Gratfreiheit kann vermieden werden, dass der Übergang vom Haltedraht/von den Haltedrähten zum Draht die Innenwandung des Katheters beim Vorschieben und Zurückziehen des Bürstenkopfes übermäßig mechanisch belastet oder sogar beschädigt.

Bei einer Ausgestaltung der erfindungsgemäßen Bürste beträgt der maximale Durchmesser des Drahtes höchstens 1 mm, insbesondere höchstens 0,8 mm, insbesondere höchstens 0,6 mm, insbesondere höchstens 0,5 mm, insbesondere höchstens 0,4 mm, insbesondere höchstens 0,3 mm, insbesondere höchstens 0,2 , insbesondere höchsten 0,1 mm. Dadurch ist es möglich, auch Katheter zu reinigen, die auf Grund ihres geringen Innendurchmessers mit bisherigen Bürsten nicht zu reinigen waren. Der maximale Durchmesser des Bürstenkopfes kann höchstens 1,2 mm, insbesondere höchstens 1,1 mm, insbesondere höchstens 1 mm, insbesondere höchstens 0,9 mm, insbesondere höchstens 0,8 mm, insbesondere höchstens 0,7 mm, insbesondere höchstens 0,6 mm, insbesondere höchstens 0,5 mm, insbesondere höchstens 0,4 mm, betragen. Der maximale Durchmesser des Bürstenkopfes ist dabei stets etwas größer als der Innendurchmesser des zu reinigenden Katheters. Der maximale Durchmesser des Bürstenkopfes wird stets durch die Fasern des Bürstenbesatzes bestimmt.

Bei einer Ausgestaltung der Bürste bestehen der Draht und der Haltedraht/die Haltedrähte jeweils unabhängig voneinander aus einem Edelstahl. Der Edelstahl kann eine Beschichtung aufweisen, die beim Anschweißen an der Schweißstelle abgeschmolzen wird. Der Draht und der Haltedraht/die Haltedrähte können auch aus demselben Edelstahl bestehen. Der Edelstahl kann beispielsweise ein Stahl mit der Werkstoffnummer 1.4301 gemäß europäischer Normung sein. Es handelt sich dabei um die Legierung X5CrNi18-10, die auch unter der Bezeichnung V2A vertrieben wird. Alternativ kann es sich bei dem Edelstahl auch um einen Stahl mit der Werkstoffnummer 1.4310 gemäß europäischer Normung handeln. Weitere geeignete Edelstähle weisen die Werkstoffnummern 1.4303, 1.4401, 1.4404 und 1.4571 gemäß europäischer Normung auf.

Durch die Verwendung von Edelstahl wird eine lange Haltbarkeit gewährleistet. Darüber hinaus bilden sich auf Edelstahl keine oder keine wesentlichen Korrosionsstellen, die beim Reinigungsprozess zu einer mechanischen Belastung durch Kratzen auf der Innenwand des Katheters führen könnten.

Zur mechanischen Schonung der Innenwand des Katheters kann der gedrehte Bereich des gedrehten Haltedrahtes/der gedrehten Haltedrähte auf seiner nicht am Draht angeschweißten Seite entgratet oder rundgeschmolzen sein. Alternativ kann darauf auch ein rundes Metallstück mit einem geringeren Außendurchmesser als dem maximalen Durchmesser des Bürstenbesatzes aufgeschweißt sein. Das Metallstück kann dabei aus einem Edelstahl, insbesondere demselben Edelstahl wie der Haltedraht, bestehen.

Bei einer Ausgestaltung der erfindungsgemäßen Bürste beträgt der geringste Abstand zwischen dem Bürstenbesatz und einem Bereich, an welchem der Haltedraht/die Haltedrähte entgratet oder rundgeschmolzen ist/sind oder dem aufgeschweißten runden Metallstück höchstens 6 mm, insbesondere höchstens 5 mm, insbesondere höchstens 4 mm, insbesondere höchstens 3 mm, insbesondere höchstens 2 mm, insbesondere höchstens 1 mm. Je geringer der genannte Abstand ist, desto geringer ist der Krümmungsradius eines gekrümmten flexiblen Katheters, welcher mit der Bürste noch gereinigt werden kann. Ein solch geringer Abstand kann dadurch erreicht werden, dass beim Schweißen eine gute Wärmeableitung vom Haltedraht/von den Haltedrähten gewährleistet wird.

Bei dem Katheter kann es sich um einen Katheter in einem medizinischen Endoskop handeln. Bei einer Ausgestaltung der erfindungsgemäßen Bürste bestehen die Fasern aus einem Polyamid, insbesondere einem Polyamid, welches antibakterielle wirkt und kein Wasser aufnimmt und damit für die vorgesehene Innenreinigung von flexiblen Kathetern besonders gut geeignet ist. Ein solches antibakteriell wirkendes und kein Wasser aufnehmendes Polyamid ist beispielsweise das Polyamid mit der allgemeinen Kurzbezeichnung PA 6.12. Bei PA 6.12 handelt es sich um Poly-(hexamethylendodecandiamid).

Nachfolgend wird die Erfindung anhand eines Ausführungsbeispiels und der Zeichnungen näher erläutert. Es zeigen:
- Fig. 1: eine erfindungsgemäße Bürste,
- Fig. 2: den Bürstenkopf mit dem gedrehten Bereich und dem Bürstenbesatz und
- Fig.3: einen alternativ ausgestalteten Bürstenkopf mit einem von nur einem Haltedraht gebildeten gedrehten Bereich und dem Bürstenbesatz.

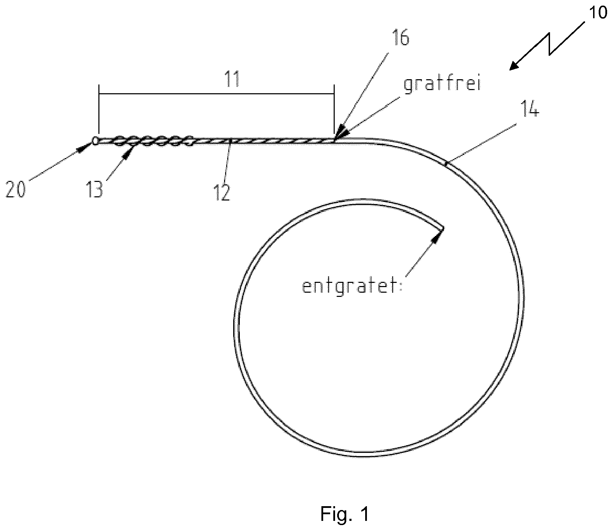

Fig. 1 zeigt eine schematische Darstellung der erfindungsgemäßen Bürste 10 mit einem gedrehten Bereich 11 zweier Haltedrähte 12, vom denen die Fasern 13 gehalten werden. Der gedrehte Bereich 11 ist an der Schweißstelle 16 gratfrei an ein Ende des Drahtes 14 angeschweißt. Am anderen Ende des Drahtes 14 ist dieser zur besseren Handhabung entgratet. Die nicht an den Draht 14 angeschweißte Seite der Haltedrähte 12 bildet das rundgeschmolzene Ende 20.

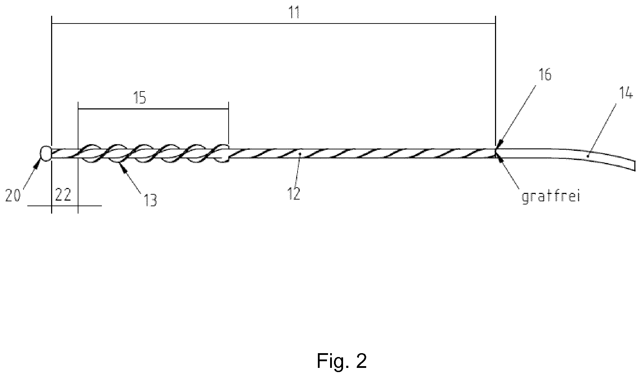

Fig. 2 zeigt eine Detaildarstellung des in Fig. 1 dargestellten gedrehten Bereichs 11 und eines kurzen Stückes des Drahtes 14. Der gedrehte Bereich 11 ist aus zwei verdrehten Haltedrähten 12 gebildet. Die durch die Haltedrähte 12 gehaltenen Fasern 13 bilden den Bürstenbesatz 15. Am der Schweißstelle 16 gegenüberliegenden Ende des gedrehten Bereichs 11 sind die beiden Haltedrähte rundgeschmolzen. Das Rundschmelzen kann beispielsweise mittels eines Laserstrahls erfolgen. Bei dem Rundschmelzen verbinden sich die beiden Haltedrähte 12 und es bildet sich ein kugelkopfartiges Ende, welches keine Beschädigungen im Inneren des zu reinigenden Katheters verursachen kann. Der Zwischenbereich 22 zwischen dem Bürstenbesatz 15 und dem Bereich, an welchem die Haltedrähte rundgeschmolzen sind, sollte möglichst kurz sein. Je kürzer dieser Zwischenbereich 22 ist, desto kleiner können die Krümmungsradien eines von der Bürste zu reinigenden Katheters sein.

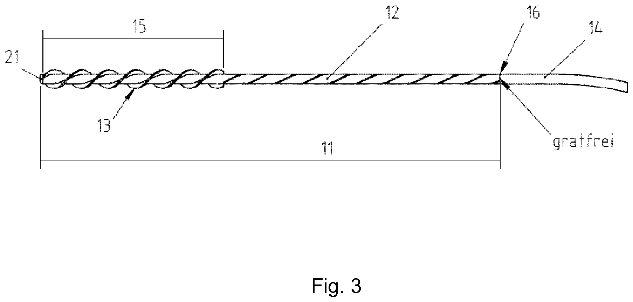

Fig. 3 zeigt eine alternative Ausgestaltung des gedrehten Bereichs 11 und eines kurzen Stückes des Drahtes 14, bei welcher der gedrehte Bereich 11 aus einem einzigen Haltedraht 12 gebildet ist, der in der Mitte umgefalzt ist, so dass an der umgefalzten Stelle eine kleine stumpfe Öse 21 gebildet ist, die ebenfalls keine Beschädigungen im Inneren des Katheters verursachen kann.

### Bezugszeichenliste

- 10: Bürste
- 11: gedrehter Bereich
- 12: Haltedraht
- 13: Fasern
- 14: Draht
- 15: Bürstenbesatz
- 16: Schweißstelle
- 20: rundgeschmolzenes Ende
- 21: Öse
- 22: Zwischenbereich

## Patentansprüche

1. Bürste (10) zur Innenreinigung eines flexiblen Katheters, wobei die Bürste (10) einen Bürstenkopf und einen länglichen flexiblen Bürstenkopfhalter zum Vorschieben und Zurückziehen des Bürstenkopfes (11) in dem Katheter aufweist, wobei der Bürstenkopf einen gedrehten Haltedraht (12) oder eine Mehrzahl gedrehter Haltedrähte (12) und Fasern (13) umfasst, wobei die Fasern von dem Haltedraht (12) oder den Haltedrähten (12) gehalten werden und einen Bürstenbesatz (15) bilden, **dadurch gekennzeichnet, dass** der Bürstenkopfhalter aus nur einem flexiblen, ungedrehten Draht (14) besteht, an dessen einem Ende der Haltedraht (12) oder die Haltedrähte (12) stumpf angeschweißt ist/sind, wobei ein Übergang vom Haltedraht/von den Haltedrähten zum Draht (14) gratfrei ist und/oder wobei ein maximaler Gesamtaußendurchmesser eines gedrehten Bereichs (11) des gedrehten Haltedrahtes (12) oder der gedrehten Haltedrähte (12) vom Außendurchmesser des Drahtes (14) gar nicht oder um höchstens 0,1 mm abweicht, wobei der maximale Durchmesser des Drahtes (14) höchstens 1 mm beträgt.

2. Bürste (10) nach Anspruch 1, **dadurch gekennzeichnet, dass** ein maximaler Gesamtaußendurchmesser eines gedrehten Bereichs (11) des gedrehten Haltedrahtes (12) oder der gedrehten Haltedrähte (12) vom Außendurchmesser des Drahtes (14) um höchstens 0,075 mm, 0,05 mm, 0,025 mm oder 0,01 mm abweicht.

3. Bürste (10) nach einem der vorhergehenden Ansprüche, **dadurch gekennzeichnet, dass** der maximale Durchmesser des Drahtes höchstens 0,8 mm, 0,6 mm, 0,5 mm, 0,4 mm, 0,3 mm, 0,2 mm oder 0,1 mm beträgt.

4. Bürste (10) nach einem der vorhergehenden Ansprüche, **dadurch gekennzeichnet, dass** der maximale Durchmesser des Bürstenkopfes (11) höchstens 1,2 mm, 1,1 mm, 1 mm, 0,9 mm, 0,8 mm, 0,7 mm, 0,6 mm, 0,5 mm oder 0,4 mm beträgt.

5. Bürste (10) nach einem der vorhergehenden Ansprüche, **dadurch gekennzeichnet, dass** der Draht (14) und der Haltedraht (12) oder die Haltedrähte (12) jeweils unabhängig voneinander aus einem Edelstahl bestehen oder der Draht und der Haltedraht (12) oder die Haltedrähte (12) aus demselben Edelstahl bestehen.

6. Bürste (10) nach einem der vorhergehenden Ansprüche, **dadurch gekennzeichnet, dass** der gedrehte Bereich (11) des gedrehten Haltedrahtes (12)/der gedrehten Haltedrähte (12) auf seiner nicht am Draht (14) angeschweißten Seite entgratet oder rundgeschmolzen ist oder darauf ein rundes Metallstück mit einem geringeren Außendurchmesser als der maximale Durchmesser des Bürstenkopfes (11) aufgeschweißt ist.

7. Bürste (10) nach Anspruch 6, **dadurch gekennzeichnet, dass** das Metallstück aus einem oder demselben Edelstahl wie der Haltedraht (12)/die Haltedrähte (12) besteht.

8. Bürste (10) nach Anspruch 6 oder 7, **dadurch gekennzeichnet, dass** der geringste Abstand zwischen dem Bürstenbesatz (15) und einem Bereich, an welchem der Haltedraht (12)/die Haltedrähte (12) entgratet oder rundgeschmolzen ist/sind oder dem aufgeschweißten runden Metallstück höchstens 6 mm, 5 mm, 4 mm, 3 mm, 2 mm oder 1 mm beträgt.

9. Bürste (10) nach einem der vorhergehenden Ansprüche, **dadurch gekennzeichnet, dass** der Katheter ein Katheter in einem medizinischen Endoskop ist.

10. Bürste (10) nach einem der vorhergehenden Ansprüche, **dadurch gekennzeichnet, dass** die Fasern (13) aus einem Polyamid bestehen.

## Claims

1. Brush (10) for cleaning the inside of a flexible catheter, wherein the brush (10) has a brush head and an elongated flexible brush head holder for advancing and retracting the brush head (11) in the catheter, wherein the brush head comprises a twisted retaining wire (12) or a plurality of twisted retaining wires (12) and fibers (13), wherein the fibers are held by the retaining wire (12) or the retaining wires (12) and form a brush filling (15), **characterized in that** the brush head holder consists of only one flexible, untwisted wire (14), on the one end thereof the retaining wire (12) or the retaining wires (12) is/are butt welded thereon, wherein a transition from the retaining wire or from the retaining wires to the wire (14) is free of burrs and/or wherein a maximum total outside diameter of a twisted region (11) of the twisted retaining wire (12) or the twisted retaining wires (12) does not deviate from the outside diameter of the wire (14) at all or deviates by at most 0.1 mm, wherein the maximum diameter of the wire (14) is at most 1 mm.

2. Brush (10) according to Claim 1, **characterized in that** a maximum total outside diameter of a twisted region (11) of the twisted retaining wire (12) or of the twisted retaining wires (12) deviates from the outside diameter of the wire (14) by at most 0.075 mm, 0.05 mm, 0.025 mm or 0.01 mm.

3. Brush (10) according to one of the preceding claims, **characterized in that** the maximum diameter of the wire is at most 0.8 mm, 0.6 mm, 0.5 mm, 0.4 mm, 0.3 mm, 0.2 mm or 0.1 mm.

4. Brush (10) according to one of the preceding claims, **characterized in that** the maximum diameter of the brush head (11) is at most 1.2 mm, 1.1 mm, 1 mm, 0.9 mm, 0.8 mm, 0.7 mm, 0.6 mm, 0.5 mm or 0.4 mm.

5. Brush (10) according to one of the preceding claims, **characterized in that** the wire (14) and the retaining wire (12) or the retaining wires (12) each consist independent of one another of a stainless steel or the wire and the retaining wire (12) or the retaining wires (12) consist of the same stainless steel.

6. Brush (10) according to one of the preceding claims, **characterized in that** the side of the twisted region (11) of the twisted retaining wire (12)/of the twisted retaining wires (12) that is not welded onto the wire (14) is deburred or rounded off by melting or a round metal piece with a smaller outside diameter than the maximum diameter of the brush head (11) is welded thereon.

7. Brush (10) according to claim 6, **characterized in that** the metal piece consists of one or the same stainless steel as the retaining wire (12) or the retaining wires (12).

8. Brush (10) according to Claim 6 or 7, **characterized in that** the smallest distance between the brush filling (15) and a region, on which the retaining wire (12)/the retaining wires (12) is/are deburred or rounded off by melting or the welded-on round metal piece is at most 6 mm, 5 mm, 4 mm, 3 mm, 2 mm or 1 mm.

9. Brush (10) according to one of the preceding claims, **characterized in that** the catheter is a catheter in a medical endoscope.

10. Brush (10) according to one of the preceding claims, **characterized in that** the fibers (13) consist of a polyamide.

## Revendications

1. Brosse (10) pour le nettoyage interne d'un cathéter flexible, dans laquelle la brosse (10) présente une tête de brosse et un support de tête de brosse flexible longitudinal pour l'avance et le retrait de la tête de brosse (11) dans le cathéter, dans laquelle la tête de brosse comprend un fil de retenue torsadé (12) ou une pluralité de fils de retenue torsadés (12) et des fibres (13), dans laquelle les fibres sont retenues par le fil de retenue (12) ou les fils de retenue (12) et forment une garniture de brosse (15), **caractérisée en ce que** le support de tête de brosse consiste en un seul fil flexible non torsadé (14) à une extrémité duquel le fil de retenue (12) ou les fils de retenue (12) est/sont soudé(s) bout à bout, dans laquelle une transition du fil de retenue/des fils de retenue au fil (14) est exempte de bavure et/ou dans laquelle un diamètre externe total maximal d'une région torsadée (11) du fil de retenue torsadé (12) ou des fils de retenue torsadés (12) ne diffère pas du tout ou de 0,1 mm maximum du diamètre externe du fil (14), dans laquelle le diamètre maximal du fil (14) se monte à 1 mm maximum.

2. Brosse (10) selon la revendication 1, **caractérisée en ce qu'**un diamètre externe total maximal d'une région torsadée (11) du fil de retenue torsadé (12) ou des fils de retenue torsadés (12) diffère de 0,075 mm, 0,05 mm, 0,025 mm ou 0,01 mm maximum du diamètre externe du fil (14).

3. Brosse (10) selon une des revendications précédentes, **caractérisée en ce que** le diamètre maximal du fil se monte à 0,8 mm, 0,6 mm, 0,5 mm, 0,4 mm, 0,3 mm, 0,2 mm ou 0,1 mm maximum.

4. Brosse (10) selon une des revendications précédentes, **caractérisée en ce que** le diamètre maximal de la tête de brosse (11) se monte à 1,2 mm, 1,1 mm, 1 mm, 0,9 mm, 0,8 mm, 0,7 mm, 0,6 mm, 0,5 mm ou 0,4 mm maximum.

5. Brosse (10) selon une des revendications précédentes, **caractérisée en ce que** le fil (14) et le fil de retenue (12) ou les fils de retenue (12) consistent chacun indépendamment les uns des autres en un acier spécial ou le fil (14) et le fil de retenue (12) ou les fils de retenue (12) consistent en le même acier spécial.

6. Brosse (10) selon une des revendications précédentes, **caractérisée en ce que** la région torsadée (11) du fil de retenue torsadé (12)/des fils de retenue torsadés (12) est ébavurée ou arrondie par fusion sur son côté non soudé au fil (14) ou une pièce métallique ronde avec un diamètre externe inférieur au diamètre maximal de la tête de brosse (11) est soudée sur celle-ci.

7. Brosse (10) selon la revendication 6, **caractérisée en ce que** la pièce métallique consiste en un ou en le même acier spécial que le fil de retenue (12)/les fils de retenue (12).

8. Brosse (10) selon la revendication 6 ou 7, **caractérisée en ce que** l'écart le plus faible entre la garniture de brosse (15) et une région sur laquelle le fil de retenue (12)/les fils de retenue (12) est/sont ébavuré(s) ou arrondi(s) par fusion ou la pièce métallique ronde soudée se monte à 6 mm, 5 mm, 4 mm, 3 mm, 2 mm ou 1 mm maximum.

9. Brosse (10) selon une des revendications précédentes, **caractérisée en ce que** le cathéter est un cathéter dans un endoscope médical.

10. Brosse (10) selon une des revendications précédentes, **caractérisée en ce que** les fibres (13) consistent en un polyamide.
